(19) Europäisches Patentamt
European Patent Office
Office européen des brevets

(11) Veröffentlichungsnummer: **0 147 778**
**A2**

# (12) EUROPÄISCHE PATENTANMELDUNG

(21) Anmeldenummer: 84115774.6

(22) Anmeldetag: 19.12.84

(51) Int. Cl.⁴: C 07 C 50/32
C 07 C 143/80, C 07 C 103/42
C 07 C 149/40, C 07 C 149/41

(30) Priorität: 31.12.83 DE 3347658

(43) Veröffentlichungstag der Anmeldung:
10.07.85 Patentblatt 85/28

(84) Benannte Vertragsstaaten:
BE CH DE FR GB IT LI NL SE

(71) Anmelder: Troponwerke GmbH & Co. KG
Berliner Strasse 156
D-5000 Köln 80(DE)

(72) Erfinder: Opitz, Wolfgang, Dr.
Holzbachtalstrasse 60
D-5063 Overath(DE)

(72) Erfinder: Pelster, Bernhard, Dr.
Pleisufer 6a
D-5205 St. Augustin 1(DE)

(72) Erfinder: Fruchtmann Romania
Konrad-Adenauer-Ufer 79/81
D-5000 Köln 1(DE)

(72) Erfinder: Krupka, Udo, Dr.
Oberer Eichweg 29
D-3550 Marburg(DE)

(72) Erfinder: Gauss, Walter, Dr.
Ludwig-Aschoff-Strasse 4
D-5000 Köln 80(DE)

(72) Erfinder: Kiehne, Hartmut, Dr.
Feld 26
D-5068 Odenthal(DE)

(72) Erfinder: Oediger, Hermann, Dr.
Roggendorfstrasse 51
D-5000 Köln 80(DE)

(74) Vertreter: Adrian, Albert, Dr. et al,
c/o BAYER AG Konzernverwaltung RP Patentabteilung
D-5090 Leverkusen 1, Bayerwerk(DE)

## (54) 1,4-Naphthochinonderivate mit entzündungshemmender Wirkung.

(57) Die 1,4-Naphthochinonderivate entsprechen der allgemeinen Formel

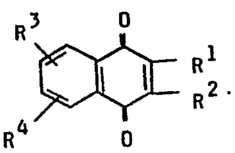

worin

$R^1$ für ein Halogenatom oder eine freie oder substituierte Hydroxyl-, Sulfhydryl- oder Amino-Gruppe stehen kann,

$R^2$ die gleiche Bedeutung wie $R^1$ hat und gleich oder von diesem verschieden sein kann oder für einen Alkylrest oder ein Wasserstoffatom stehen kann und

$R^3$ und $R^4$ gleich oder verschieden sind und Wasserstoff, Alkyl, Hydroxyl, Alkoxy, Sulfonamid oder Halogen bedeuten können, wobei für den Fall, daß $R^1$ Hydroxyl bedeutet, die Reste $R^2$, $R^3$ und $R^4$ Wasserstoff bedeuten, und können als Wirkstoffe in Arzneimitteln verwendet werden.

EP 0 147 778 A2

TROPONWERKE GmbH & Co. KG

5000 Köln 80
Je/jd-c

1.4-Naphthochinonderivate mit entzündungshemmender Wirkung

Gegenstand der vorliegenden Erfindung sin Naphthochinonderivate sowie deren Verwendung als aktive Komponenten in entzündungshemmenden Arzneimitteln.

Die erfindungsgemäßen Naphthochinonderivate entsprechen der allgemeinen Formel I

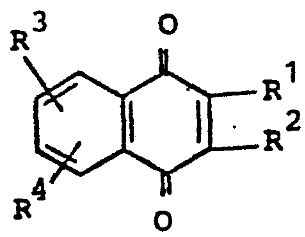

(I)

worin

$R^1$ für ein Halogenatom oder eine freie oder substituierte Hydroxyl-, Sulfhydryl- oder Amino-Gruppe stehen kann,

$R^2$ die gleiche Bedeutung wie $R^1$ hat und gleich oder von diesem verschieden sein kann oder für einen Alkylrest oder ein Wasserstoffatom stehen kann und

$R^3$ und $R^4$ gleich oder verschieden sind und Wasserstoff, Alkyl, Hydroxyl, Alkoxy, Sulfonamid oder Halogenbedeuten können, wobei für den Fall, daß $R^1$ Hydroxyl bedeutet, die Reste $R^2$, $R^3$ und $R^4$ Wasserstoff bedeuten.

Überraschenderweise wurde gefunden, daß die erfindungsgemäßen 1,4-Naphthochinonderivate hinsichtlich ihrer entzündungshemmenden und antiarthritischen Wirkungen überlegene Wirkungen gegenüber vergleichbaren Wirkstoffen zeigen, da sie bei allen üblichen Applikationsformen bei geringer Allgemeintoxizität ausgezeichnet entzündungshemmend und antiarthritisch wirken.

Die erfindungsgemäßen 1,4-Naphthochinonderivate sind teilweise neu.

Gegenstand der vorliegenden Erfindung sind daher auch neue Verbindungen der allgemeinen Formel II

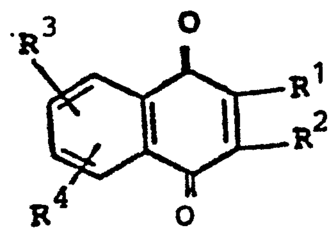

(II)

worin

$R^1$ Alkylamino-, Acetyl- oder Propionylaminorest,

$R^2$ Wasserstoff, 2-Methoxy-ethoxy- oder Ethoxyrest,

$R^3$ Wasserstoff oder einen Sulfamoylrest und

$R^4$ Wasserstoff bedeutet.

Bevorzugte Verbindungen der allgemeinen Formel II sind 2-Allylamino-6-sulfamoyl-naphthochinon-1.4, 2-Propionylamino-3-(2-methoxyethoxy)-naphthochinon-1.4 und 2-Acetylamino-3-ethoxy-naphthochinon-1.4.

Bevorzugte 1,4-Naphthochinonderivate der allgemeinen Formel I sind solche, worin

$R^1$ für eine Hydroxylgruppe,
für eine Alkoxygruppe mit ein bis zwei C-Atomen, die durch eine Methoxygruppe substituiert sein kann,

für eine Carboxymethylmercaptogruppe und davon abgeleitete Alkylamid- und Hydroxylalkylamidderivate, deren Alkylreste bis zu drei C-Atome enthalten kann,

für eine freie oder eine gesättigte oder ungesättigte Monoalkylaminogruppe mit bis zu 13 C-Atomen, die durch eine Hydroxyl- oder eine Acetoxygruppe substituiert sein kann,

für eine Phenylaminogruppe, die durch ein oder zwei Halogenatome, bevorzugt Chloratome, eine Methoxy-, Nitro-, Carboxyl- oder Sulfamoylgruppe, substituiert sein kann,

für eine Acetyl- oder Propionylaminogruppe oder ein Chloratom stehen kann,

$R^2$ die gleiche Bedeutung wie $R^1$ hat und gleich oder von diesem verschieden sein kann oder für eine Methyl- oder Ethylgruppe oder ein Wasserstoffatom stehen kann und

$R^3$ und $R^4$, die gleich oder verschieden sind und für Wasserstoff, $C_1$-$C_4$-Alkyl, Hydroxyl, $C_1$-$C_4$-Alkoxy, Sulfonamid oder Halogen stehen können.

Besonders bevorzugte Verbindungen der allgemeinen Formel I sind:

Verb. 1: 2-Hydroxynaphthochinon-1.4

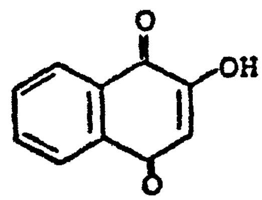

Verb. 2: 2-Methoxynaphthochinon-1.4

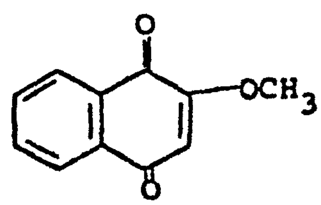

Verb. 3: 2-(2-Methoxyethoxy)-naphthochinon-1.4

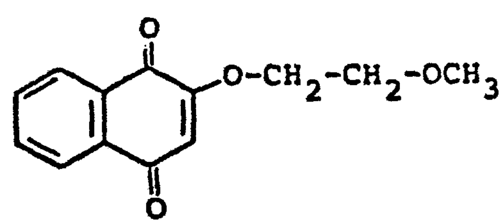

TP 70

Verb. 4: 2.3-Dimethoxynaphthochinon-1.4

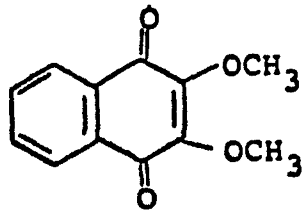

Verb. 5: 2-Aminonaphthochinon-1.4

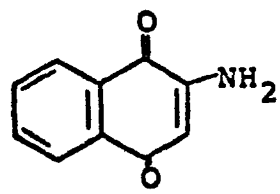

Verb. 6: 2-Amino-6-sulfamoylnaphthochinon-1.4

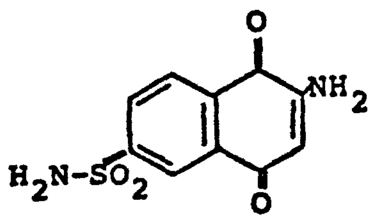

Verb. 7: 2-N-Acetylaminonaphthochinon-1.4

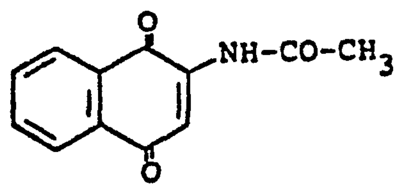

Verb. 8: 2-Methyl-3-carboxymethylmercaptonaphthochinon-1.4

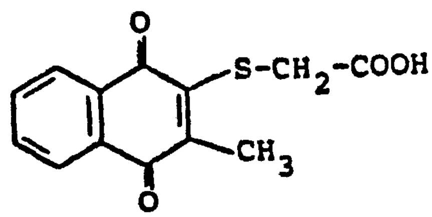

Verb. 9: 2-Methyl-3-methylaminocarbonylmethylmercaptonaphthochinon-1.4

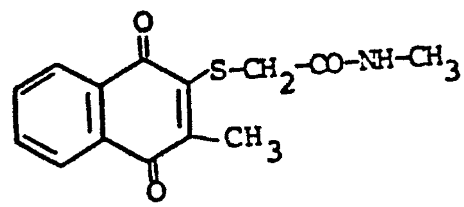

Verb. 10: 2-Methyl-3-isopropylaminocarbonylmethylmercaptonaphthochinon-1.4

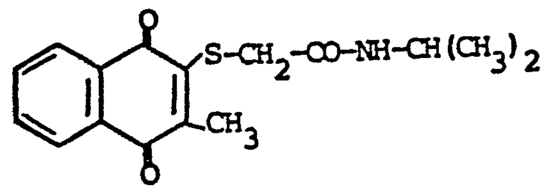

Verb. 11: 2.3-Bis(2-hydroxyethylaminocarbonylmethylmercapto)-naphthochinon-1.4

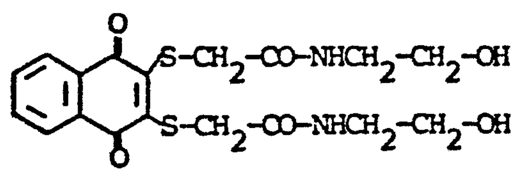

sowie die nachfolgend aufgeführten neuen Verbindungen, die als solche erfindungsgemäß besonders bevorzugt sind.

Verb. 12: 2-Allylamino-6-sulfamoylnaphthochinon-1.4

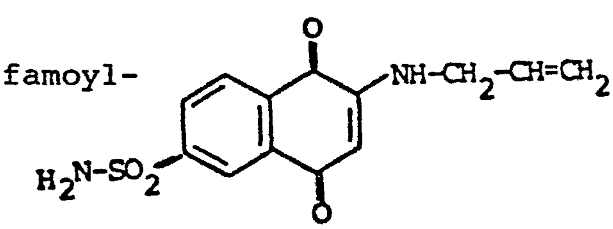

Verb. 13: 2-Propionylamino-3-(2-methoxyethoxy)-naphthochinon-1.4

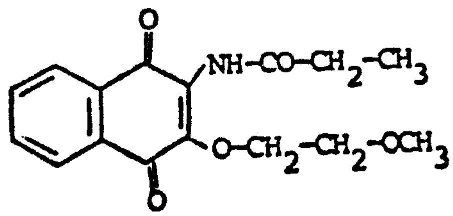

Verb. 14: 2-Acetylamino-3-ethoxy-naphthochinon-1.4

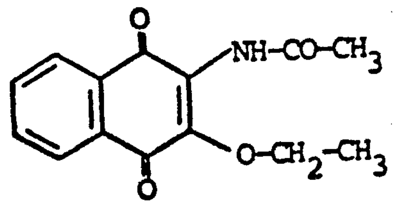

Die bekannten 1,4-Naphthochinonderivate können nach an sich bekannten Verfahren hergestellt werden. So wird beispielsweise die Herstellung von 2-Methyl-3-carboxymethylmercaptonaphthochinon-1,4 (Verb.8), 2-Methyl-3-isopropylaminocarbonylmethylmercaptonaphthochinon-1,4 (Verb. 10) in Liebigs Annalen der Chemie 758, 1ff (1972) beschrieben.

Die Herstellung von 2-(2-Methoxyethoxy)-naphthochinon-1,4 (Verb. 3) wird in den DE-Os 25 16 270, US 41 95 998 und US 42 01 588 beschrieben. 2,3-Bis-(2-hydroxyethyl-aminocarbonylmethylmercapto)-naphthochinon-1,4 (Verb. 11) ist aus der DE-OS 19 10 894 bekannt.

Die neuen Verbindungen können beispielsweise aus einem Chlor-acetylaminonaphthochinon-1,4 durch Umsetzung mit einem entsprechenden Alkohol bei Raumtemperatur (0 bis 40°C) hergestellt werden.

Die erfindungsgemäßen 1,4-Naphthochinonderivate sind Wirkstoffe für Arzneimittel. Im besonderen sind sie wirksam bei der Bekämpfung von Entzündungen und der Bekämpfung von rheumatischen Erkrankungen.

Bevorzugte Wirkstoffe sind 2-Methyl-3-carboxymethyl-mercapto-naphthochinon-1,4, 2 Methyl-3-methyl-mercapto-naphthochinon-1,4 und 2-Methyl-3-isopropyl-aminocarbonylmethylmercapto-naphthochinon-1,4.

Die erfindungsgemäßen 1,4-Naphthochinonderivate hemmen das durch Carrageenan ausgelöste Ödem der Rattenpfote überraschend stark.

Die erfindungsgemäßen 1,4-Naphthochinonderivate hemmen ebenso die Metabolisierung der Arachidonsäure über den Cyclooxygenaseweg. (Methode von Brune et al., Naunyn-Schmiedeberg's Arch. Pharmacol. 315, 269 (1981)).

Überraschenderweise hemmen darüber hinaus die erfindungsgemäßen 1,4-Naphthochinonderivate auch die Metabolisierung der Arachidonsäure über den Lipoxygenaseweg außerordentlich stark.

Darüber hinaus konnte für die erfindungsgemäßen 1,4-Naphthochinonderivate nachgewiesen werden, daß sie die durch das chemotaktisch wirkende $LTB_4$ stimulierte Aggregation von PMN hemmen und dadurch Anteil an der entzündungshemmenden Wirkung haben (Methode von Cunningham et al., Agents & Actions 11, 583 (1981)).

Zur vorliegenden Erfindung gehören pharmazeutische Zubereitungen, die neben nichttoxischen, inerten, pharmazeutisch geeigneten Trägerstoffen einen oder mehrere erfindungsgemäße Wirkstoffe enthalten, oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie Füll- und Streckmittel (z. B. Stärken, Milchzucker, Rohrzucker, Glukose, Mannit und Kieselsäure), Bindemittel (z. B. Carboxymethylcellulose, Alginate, Gelatine, Polyvinylpyrrolidon), Feuchthaltemittel (z. B. Glycerin), Sprengmittel (z. B. Agar-Agar, Calciumcarbonat und Natriumbicarbonat), Lösungsverzögerer (z. B. Paraffin) und Resorptionsbeschleuniger (z. B. quarternäre Ammoniumverbindungen), Adsorptionsmittel (z. B. Kaolin und Bentonit) und Gleitmittel (z. B. Talkum, Calcium- und Magnesiumstearat und feste Polyethylenglykole) oder Gemische der aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert, abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen zur Erzielung eines Retardeffektes.

Suppositorien können neben dem oder den Wirkstoffen die üblichen wasserlöslichen oder wasserunlöslichen Trägerstoffe enthalten, z. B. Polyethylenglykole, Fette z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten (z. B. tierische und pflanzliche Fette, Wachse, Paraffine, Stärken, Tragant) oder Gemische dieser Stoffe.

Puder und Sprays können neben dem oder den Wirkstoffen die üblichen Trägerstoffe enthalten (z. B. Milchzucker, Talkum, Kieselsäure, Aluminiumhydroxid) oder Gemische dieser Stoffe. Sprays können zusätzlich die üblichen Treibmittel enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, Öle, insbesondere Baumwollsaatöl, Erdnußöl, Maiskeimöl, Olivenöl und Sesamöl, Glycerin, Polyethylenglykole oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel (z. B. Wasser, Ethylalkohol, Propylenglykol), Suspendiermittel (z. B. ethoxylierte Isostearylalkohole, Polyoxyethylensorbit- und Sorbitanester, mikrokristalline Cellulose) oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung entzündlicher Prozesse im menschlichen und tierischen Organismus.

Die Wirkstoffe oder die pharmazeutischen Zubereitungen können cutan, oral, parenteral, intraperitoneal und/oder rectal, vorzugsweise oral oder cutan, appliziert werden.

Im allgemeinen ist es in der Humanmedizin als vorteilhaft anzusehen, den oder die erfindungsgemäßen Wirkstoffe in Gesamtmengen von etwa 0,1 bis 200, vorzugsweise 0,1 bis 70 mg je 24 Stunden, gegebenenfalls in Form mehrerer Einzelgaben zur Erzielung der gewünschten Ergebnisse, zu verabreichen.

Es kann jedoch erforderlich sein, von den genannten Dosierungen abzuweichen, und zwar in Abhängigkeit von der Art und dem Körpergewicht des zu behandelnden Objektes, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß.

Herstellungsbeispiele:

Beispiel 1

2-Allylamino-6-sulfamoylnaphthochinon-1.4 (Verb. 12) wurde, ausgehend von 1.4-Dioxo-1.4-dihydronaphthalinsulfonsäure 6 (Beilstein 11 (III), 622), durch Überführung in das Sulfonamid und dessen anschließende Umsetzung mit Allylamin in Gegenwart von Luftsauerstoff erhalten. Fp. 222 - 23 °C.

Beispiel 2

2-Propionylamino-3-(2-methoxyethoxy)-naphthochinon-1.4 (Verb. 13)

Eine Suspension von 26.35 g (0.1 Mol) 3-Chlor-2-propionylaminonaphthochinon-1.4 in 100 ml Ethylenglykolmonomethylether wird mit 15.2 g (0.1 Mol) 1.8-Diazabicyclo (5.4.0)undec-7-en in 50 ml Ethylenglykolmonomethylether versetzt und das Gemisch über Nacht bei Raumtemperatur aufbewahrt. Nach Kühlen auf 0 °C wird filtriert und der Niederschlag mit kaltem Methanol ausgewaschen. Ausbeute: 19.3 g (64 % d. Th.), Fp. 116 - 17°C.

Beispiel 3

2-Acetylamino-3-ethoxynaphthochinon-1.4 (Verb. 14) wird in analoger Weise wie Verb. 13 aus 3-Chlor-2-acetylaminonaphthochinon-1.4 (Ber. Dtsch. Chem. Ges. 56, 1291 (1923)) und Ethanol erhalten. Ausbeute: 56 % d. Th., Fp. 155 - 56 °C.

Anwendungsbeispiele 4 bis 17:

Verb. 1: 2-Hydroxynaphtho-chinon-1.4

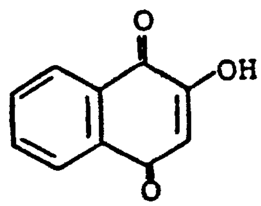

Verb. 2: 2-Methoxynaphtho-chinon-1.4

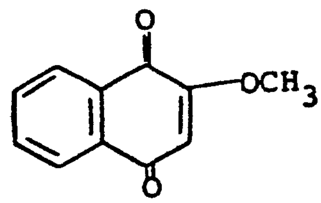

Verb. 3: 2-(2-Methoxyethoxy)-naphthochinon-1.4

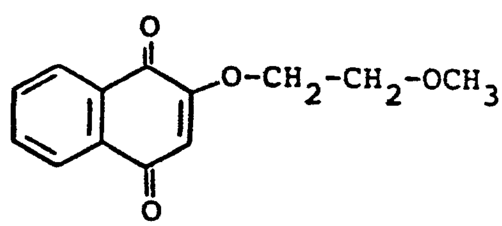

Verb. 4: 2.3-Dimethoxy-naphthochinon-1.4

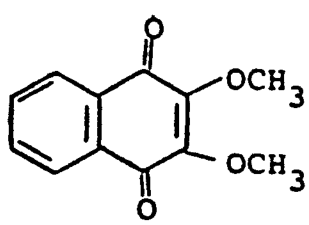

Verb. 5: 2-Aminonaphtho-
chinon 1.4

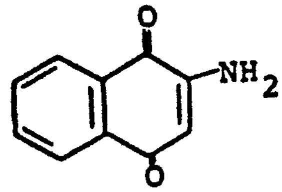

Verb. 6: 2-Amino-6-sulfamoyl-
naphthochinon-1.4

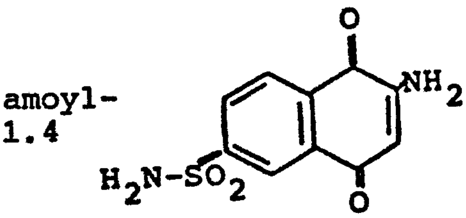

Verb. 7: 2-N-Acetylamino-
naphthochinon-1.4

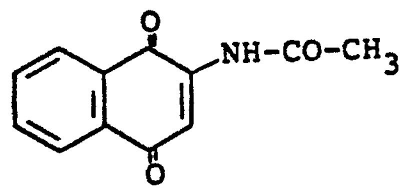

Verb. 8: 2-Methyl-3-carboxymethyl-
mercaptonaphthochinon-1.4

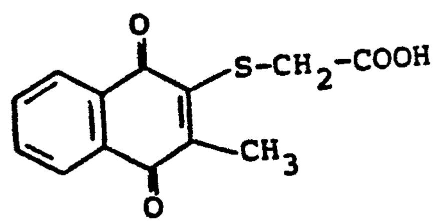

Verb. 9: 2-Methyl-3-methylamino-
carbonylmethylmercapto-
naphthochinon-1.4

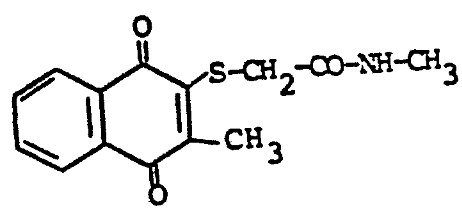

Verb. 10: 2-Methyl-3-isopropyl-aminocarbonylmethylmercaptonaphthochinon-1.4

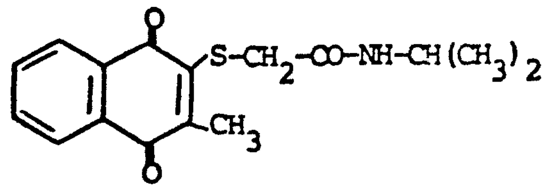

Verb. 11: 2.3-Bis(2-hydroxyethyl-aminocarbonylmethylmercapto)-naphthochinon-1.4

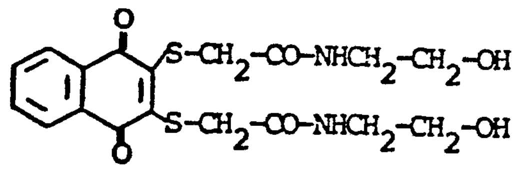

Verb. 12: 2-Allylamino-6-sulfamoyl-naphthochinon-1.4

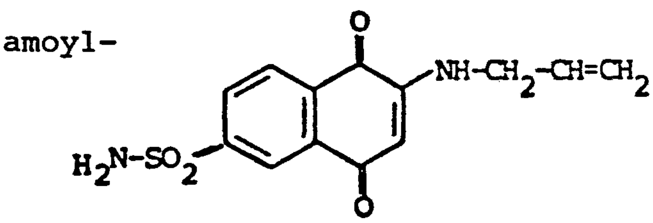

Verb. 13: 2-Propionylamino-3-(2-methoxyethoxy)-naphthochinon-1.4

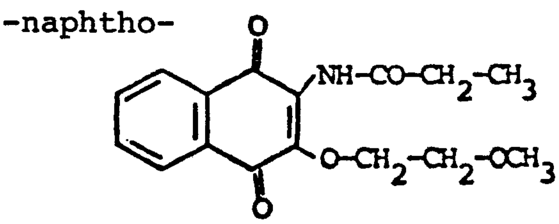

Verb. 14: 2-Acetylamino-3-ethoxy-naphthochinon-1.4

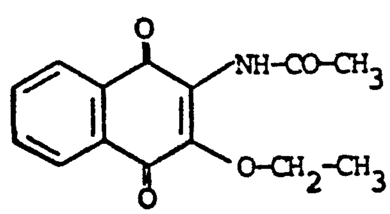

Die Verbindungen 1 bis 14 werden auf die Hemmwirkung des durch Carrageenan ausgelösten Ödems der Rattenpfote untersucht. Zum Nachweis diente die Methode von Winter et al., Proc. Soc. Exp. Biol. Med. 111, 544 (1962), mit der Abwandlung, daß zur Ödemauslösung reines -Carrageenan als 0,25 % Suspension in physiologischer Kochsalzlösung verwendet wurde (Tab. 1, Sp. 1).

Die Verbindungen 1 bis 14 wurden auf die Hemmwirkung der Metabolisierung der Arachidonsäure über den Cyclooxygenaseweg (Methode von Brune et al., Naunyn-Schmiedeberg's Arch. Pharmacol. 315, 269 (1981)) (Tab. 1, Sp. 2) untersucht.

Die Untersuchung in Tab. 1, Sp. 3, zeigt, daß die Verbindungen 1 bis 14 darüber hinaus auch die Metabolisierung der Arachidonsäure über den Lipoxygenaseweg außerordentlich stark hemmen.

Die Meßmethode beruht auf der Bestimmung der Freisetzung von $LTB_4$ aus neutrophilen Granulozyten (PMN) der Ratte nach Stimulation mit Ca-Ionophor mittels reversed phase-HPLC (Borgeat et al., Proc. Natl. Acad. Sci. USA 76, 2148 (1979), Ford-Hutchinson et al., Brit. J. Pharmacol. 76, 215 (1982).

Die entzündungshemmende Wirkung der Verbindungen 1 bis 14 wurde durch die durch das chemotaktisch wirkende $LTB_4$ stimulierte Aggregationshemmung von PMN (Methode von Cunningham et al., Agents & Actions 11, 583 (1981) untersucht (Tab. 1, Sp. 4).

TP 70

| Beisp. | Verb. | % Hemmung des Carrageen.-Ödems bei 2,5 mg/kg p.o. | % Hemmung der Cyclooxygenase bei $1x10^{-5}$ Mol/l | % Hemmung der Lipoxygenase bei $1x10^{-5}$ Mol/l | % Hemmung der Aggregation von PMN bei $1x10^{-4}$ Mol/l |
|---|---|---|---|---|---|
| 4 | 1 | 34 | 31 | 70 | |
| 5 | 2 | 29 | 78 | 98 | 76 |
| 6 | 3 | 38 | 45 | 100 | |
| 7 | 4 | 36 | 37 | 100 | 79 |
| 8 | 5 | 48 | 55 | 100 | |
| 9 | 6 | 39 | 65 | 100 | 77 |
| 10 | 7 | 32 | 65 | 100 | 49 |
| 11 | 8 | 41 | 72 | 100 | 34 |
| 12 | 9 | 25 | 89 | 100 | 100 |
| 13 | 10 | 45 | 89 | 100 | 95 |
| 14 | 11 | 45 | 50 | 80 | 36 |
| 15 | 12 | 39 | 38 | 100 | 72 |
| 16 | 13 | 48 | | 100 | |
| 17 | 14 | 49 | | 100 | |

Patentansprüche

1. Arzneimittel, enthaltend 1,4-Naphthochinonderivate der Formel

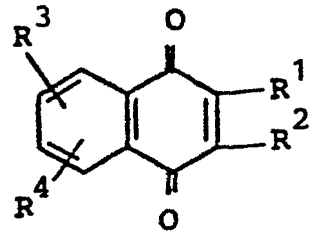

worin

$R^1$ für ein Halogenatom oder eine freie oder substituierte Hydroxyl-, Sulfhydryl- oder Amino-Gruppe stehen kann,

$R^2$ die gleiche Bedeutung wie $R^1$ hat und gleich oder von diesem verschieden sein kann oder für einen Alkylrest oder ein Wasserstoffatom stehen kann und

$R^3$ und $R^4$ gleich oder verschieden sind und Wasserstoff, Alkyl, Hydroxyl, Alkoxy, Sulfonamid oder Halogen bedeuten können,
wobei für den Fall, daß $R^1$ Hydroxyl bedeutet, die Reste $R^2$, $R^3$ und $R^4$ Wasserstoff bedeuten.

2. Verwendung von 1,4-Naphthochinonderivaten der Formel

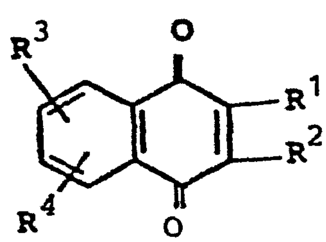

worin

$R^1$ für ein Halogenatom oder eine freie oder substituierte Hydroxyl-, Sulfhydryl- oder Amino-Gruppe stehen kann.

$R^2$ die gleiche Bedeutung wie $R^1$ hat und gleich oder von diesem verschieden sein kann oder für einen Alkylrest oder ein Wasserstoffatom stehen kann und

$R^3$ und $R^4$ gleich oder verschieden sind und Wasserstoff, Alkyl, Hydroxyl, Alkoxy, Sulfonamid oder Halogen bedeuten können, wobei für den Fall, daß $R^1$ Hydroxyl bedeutet, die Reste $R^2$, $R^3$ und $R^4$ Wasserstoff bedeuten,

zur Herstellung von Arzneimitteln.

3. Verwendung nach Anspruch 2, dadurch gekennzeichnet, daß das Arzneimittel 0,1 bis 99,5 Gew.-% des Wirkstoffs enthält.

4. Verwendung nach den Ansprüchen 2 und 3, dadurch gekennzeichnet, daß als Wirkstoff 2-Methyl-3-carbonylmethyl-mercaptonaphthochinon-1,4 eingesetzt wird.

5. Verwendung nach den Ansprüchen 2 und 3, dadurch gekennzeichnet, daß als Wirkstoff 2-Methyl-3-methylamino-carbonylmethylmercaptonaphthochinon-1,4 eingesetzt wird.

6. Verwendung nach den Ansprüchen 2 und 3, dadurch gekennzeichnet, daß als Wirkstoff 2-Methyl-3-isopropylamino-carbonylmethyl-mercapto-naphthochinon-1,4 eingesetzt wird.

7. Verwendung von Arzneimitteln nach Anspruch 1 zur Bekämpfung von Entzündungen.

8. Verwendung von Arzneimitteln nach Anspruch 1 zur Bekämpfung von rheumatischen Erkrankungen.

9. Neue 1,4-Naphthochinonderivate der Formel

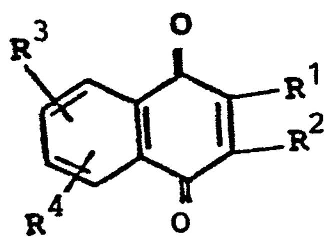

(II)

$R^1$ Alkylamino-, Acetyl- oder Propionglaminorest,

$R^2$ Wasserstoff, 2-Methoxy-ethoxy- oder Ethoxyrest,

$R^3$ Wasserstoff oder einen Sulfamoylrest und

$R^4$ Wasserstoff bedeutet.